# EUROPEAN PATENT SPECIFICATION

(11) **EP 0 540 598 B1**
(45) Date of publication and mention of the grant of the patent: **13.04.1994**
(21) Application number: 91913389.2
(22) Date of filing: 24.07.1991
(51) Int. Cl.: A61F 7/00, B29D 22/00, B05D 1/14

(54) **PROCESS FOR MANUFACTURING COATED HOT WATER CONTAINERS AND CONTAINERS THUS OBTAINED**
VERFAHREN ZUR HERSTELLUNG BEDECKTEN HEISSWASSERBEHÄLTER UND SO HERGESTELLTE BEHÄLTER
PROCEDE DE FABRICATION DE RECIPIENTS D'EAU CHAUDE AYANT UN REVETEMENT ET RECIPIENTS AINSI OBTENUS

(30) Priority: 26.07.1990 IT 2107790
(43) Date of publication of application: 12.05.1993
(73) Proprietor: GISLON, Diego, I-20131 Milan (IT)
(72) Inventor: GISLON, Diego, I-20131 Milan (IT)
(74) Representative: Marietti, Giuseppe
(86) International application number: EP9101384
(87) International publication number: WO9201429

(56) References cited:
- US-A- 2 153 441
- US-A- 3 451 884
- US-A- 3 523 848
- US-A- 3 610 307
- US-A- 4 421 809
- Kunststoffe, volume ZM11, 1 April 1991, Carl Hanser Verlag (Munich, DE) U. Maag : "Flor für anspruchsvolle Oberflächen"

## Description

The present invention concerns a process for the production of water containers for domestic usage and particularly a water container made of rubber or other plastic materials, of the kind known as "hot water bottle".

Such containers are well known since long and consist of a "bag" (pouch) of flat-shape, generally made of rubber, and provided with a stopper for closing the container safely and preventing leakage of the water therein contained. As the name itself suggests, the function of hot water bottles is to emit heat: filled with hot water they give out progressively the water heat in the surrounding environment and, above all, they pass it on to the bodies with which they are in contact.

Hot water bottles are used therefore as an absolutely safe and controlled source of heat for people, in particular for people who are ill or unwell.

The main disadvantage in the use of such hot water bottles lies in having to place the bottle in close contact with a person's body with the ensuing unpleasant feeling that this physical contact causes, because of the nature of the material used to make the bottle as well as because of the excessive heat, at least at the beginning, conveyed to the user from the hot water.

In order to avoid such inconvenience various kinds of bottles have been suggested provided with coatings destined to mediate the heat transmission and to make the contact between the user and the bottle pleasanter. In particular, removable external claddings, usually in cloth, produced separately, are usually provided and these are taken off the bottle when it is filled, so as to prevent wetting the cladding and leakage of water between the cladding and the bag.

This solution, however, has resulted hardly satisfactory, both from the aestethic point of view and the necessity of being obliged every time to put the above cited cladding on and off.

Further inconvenience of the known pouches is due to the actual impossibility of decorating them or embellishing them with aestetic motifs which may render their usage pleasanter, above all to children.

US-A-2,153,441, filed 1937, discloses a process for producing hot water bottles according to which a textile bottle template is first prepared and is successively internally coated with rubber latex to make it watertight. US-A-3,610,307 discloses a somewhat similar process that provides co-vulcanization of a textile fabric with the two rubber halves of a hot water bottle. These processes have several drawbacks, in that they are both time consuming and expensive and are not suitable for known, commercially available, hot water bottles made of thermoplastic materials. Because of these drawbacks, the common way to provide a coating to hot water bottles was to stick on them a fabric, e.g. as disclosed in FR-A-2583287.

Flocking techniques were used in completely different fields of the art, e.g. as in US-A-4,421,809 and US-A-3,451,884 where flocked coatings are used to provide a surface that absorbs water containing material such as wet soil and wet concrete paste. In other words, the teaching of these documents was to use flocked coatings as means to absorb water.

One of the aims of the present invention is to solve the above mentioned problems by making a hot water bottle which is provided with a fixed coating, integral with the bag itself, which can be acceptable both aestethically and functionally.

Another aim of the invention is to obtain a process for producing the above mentioned bag in a simple, economical way that can be carried out on industrial scale.

In detail, the present invention consists in a process for producing water bags or containers for domestic usage, in particular hot water bottles, characterized in comprising the step of fixing onto at least part of the surface of said bottle a plurality of fibers with preset dimensions, according to the method known as flocking.

The invention, besides, provides a bottle or container for water for domestic usage, in particular a hot water bottle, characterized in that at least part of its external surface is provided with a coating composed of a multiplicity of fibers with pre-set dimensions and secured to said container by means of the technique known as flocking.

In a preferred embodiment the flocked bottle, or container according to the invention, is provided with coatings having different thickness on different areas of its surface.

The invention will now be described in more detail with reference to the drawings enclosed by way of example without any limiting purpose, in which:
- figure 1 is a perspective view of a possible embodiment of the invention; and
- figure 2 is a view in section of the embodiment as per figure 1.

According to the principle of the invention, the process for producing hot water bottles provides a step in which a coating consisting of a multiplicity of flock fibers with pre-set dimensions, is applied to them. These fibers, which generally are about few millimeters or tenths of millimeters long and are made of textile fibers, natural or not, are fixed to the surface of the bottle by means of a bonding agent, or through the softening of the surface itself, according to the tecnique know as flocking.

Preferably it will be used a so called electrostatic flocking technique as it allows a better alignment of the fibers in respect to the surface of the bottle and therefore a better final effect of "hand", which makes the coating more pleasant to touch. It is however possible to utilize other techniques such as the one using a spray-gun.

The choice of the kind of fibers and of the bonding agent will be evident to the skilled technician: for instance a glue of the kind normally used for the flocking of working gloves can be used, although other kinds of glue can be equally used such as the one commercialized under the trade name of PECACOL. Also, glues usually used for flocking weather strips for car windows can be used as bonding agents for hot water bottle made of same or similar plastic materials.

Of course with the term "bonding agent" it is here meant any means apt to fix the above mentioned fibres to the surface of the bag. For instance, for containers made of thermoplastic material, the surface of the container, or bottle, can be heated and superficially softened and then the flock fibers applied to the surface thus made plastic and adhesive.

These flocking techniques are well known to the skilled technician. Similarly, selecting the appropriate kind of flock fiber will be evident to the technician due to the fact that all fibers normally used for the flocking of products can be utilized. Preferably non-absorbing fibres, such as nylon and similar products, will be employed. Always keeping in mind the kind of product (i.e. hot water bottles) on which they are applied, said fibers will preferably have a reduced length so as to allow, should part of the coating be impregnated with water accidentally, a quick drying thanks to the limited amount of water withheld.

According to a preferred aspect of the invention, the coating is treated with suitable waterproofing agents, in order to make it waterrepellent.

For instance, a suitable waterproofing agent is the one sold under the name of REFLEX PROTECTOR by FABBRICA CHIMICA UNIONE Srl of Milan.

According to another preferred aspect of the invention, different fibers and/or fibers having different lenghts are applied to different areas of the bottle. In this way different characteristics and thickness of the coating can be obtained according to the bottle areas; it is thus possible to obtain a differentiated heat transmission according to the temperature of the water contained in the bottle. With a high temperature one will put in contact of the body the side of the bottle provided with a thicker coating and viceversa when the water temperature is lower (i.e. has decreased).

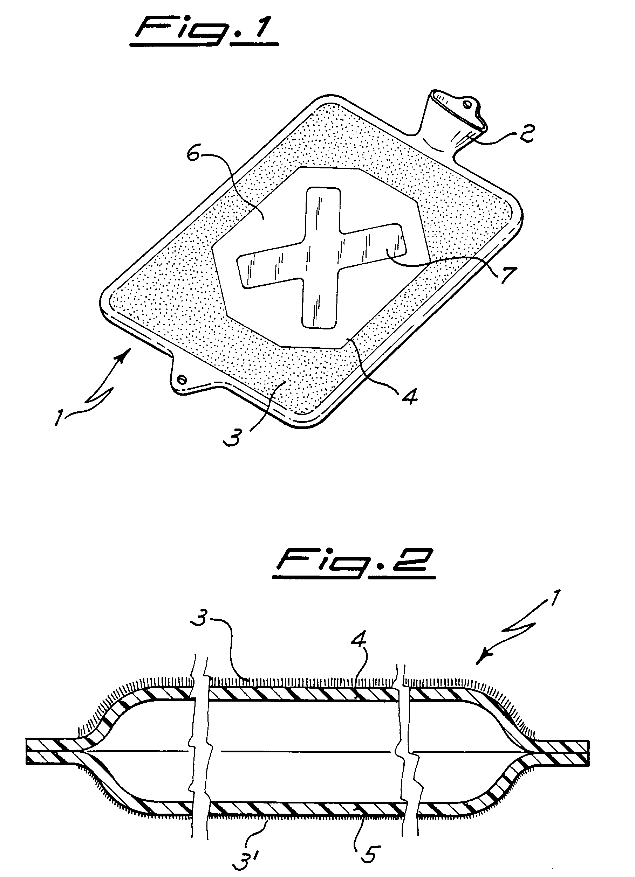

In figure 1 a perspective view of a bottle, according to the present invention is shown. As it can be observed, the bottle has the usual shape of hot water bottles, with a flat and wide structure to favour the transmission of heat and a funnel-shaped portion 2 to facilitate the filling with water. Obviously the invention application should not be limited to this single shape of hot-water bottles, as well as to rubber bottles only, as the process, according to the invention, can be applied to containers of any other kind of plastic material.

The surface of the bottle is at least partially provided with a coating 3 formed by a multiplicity of fibers having pre-set dimensions and applied to said surface by means of the technique known as flocking.

In the embodiment shown in figure 2, coating 3 on side 4 shows a different thickness from the one (3') provided on side 5 of the bottle, so as to obtain a differentiated heat transmission as previously described.

Preferably, moreover, said coating is treated with water repellent products that improve its behaviour in presence of water, avoiding the impregnation of fibers and coating. In figure 1 it is also shown a decoration of bag 1, obtained by varnishing of areas 6, 7 of coating 3. This varnishing technique on flocked surfaces is known and is commonly utilized in the production of toys; the choice of the kind of paint is not difficult therefore for the expert technician.

Alternatively, decorating effects can be obtained by selectively flocking different areas with differently coloured flock fibers.

From what above described it is evident that the invention allows to obtain hot water bottles that are pleasanter to touch than those already known and that allow optimum regulation of heat transmission from the water to the exterior.

## Claims

1. A process for the production of hot water bottles, characterized in comprising the step of coating at least part of the surface (4,5) of said bottle (1) by securing to said surface a plurality of fibers (3,3'), having preset dimensions, to at least part of the surface of said bottle, by means of the technique known as flocking.

2. A process according to claim 1, characterized in making said flocked coating (3,3') with different characteristics in different areas of said bottle.

3. A process according to claim 1 or 2, characterized in treating said fibers, before or after their application, with one or more water repellent or waterproofing products.

4. A process according to claim 1 or 2, characterized in varnishing at least part (6,7) of the above mentioned flock coating.

5. A hot water bottle (1), characterized in that at least part of its surface (4,5) is provided with a coating (3,3') composed of a multiplicity of flock fibers having pre-set dimensions and secured to said container by means of bonding agent according to the flocking technique.

6. A hot water bottle according to claim 5, characterized in that said coating (3,3') presents different thickness and/or characteristics in different areas of said bottle (1).

7. A hot water bottle according to claim 5 or 6, wherein said coating (3,3') and/or said fibers have waterrepellant properties.

8. A hot water bottle according to claim 5 or 6, wherein at least part (6,7) of said flocked coating (3,3') is varnished.

9. A hot water bottle according to any claim from 5 to 8, wherein the body of said bottle (1) is made of thermoplastic material.

10. A hot water bottle, as obtainable through a process according to any claim 1 to 4.

## Patentansprüche

1. Verfahren zur Herstellung von Wärmflaschen, gekennzeichnet durch den Schritt des Beschichtens mindestens eines Teiles der Oberfläche (4. 5) der Flasche (1) durch Befestigen einer Vielzahl von Fasern (3. 3') mit vorgegebenen Größen auf der Oberfläche auf mindestens einem Teil der Oberfläche der Flasche mittels des als Beflockung bekannten Verfahrens.

2. Verfahren nach Anspruch 1. gekennzeichnet durch Herstellen der Flockbeschichtung (3, 3') mit unterschiedlichen Eigenschaften in verschiedenen Bereichen der Flasche.

3. Verfahren nach Anspruch 1 oder 2. gekennzeichnet durch Behandeln der Fasern vor oder nach ihrem Auftragen mit einem oder mehreren wasserabstoßenden oder wasserdichten Erzeugnissen.

4. Verfahren nach Anspruch 1 oder 2. gekennzeichnet durch Lackieren mindestens eines Teiles (6. 7) der obengenannten Flockbeschichtung.

5. Wärmflasche (1), dadurch gekennzeichnet, daß mindestens ein Teil ihrer Oberfläche (4. 5) mit einer Beschichtung (3, 3') versehen ist. die aus einer Vielzahl von Flockfasern mit vorgegebenen Größen besteht und die an dem Behälter mittels eines Verbindungsmittels entsprechend dem Beflockungsverfahren befestigt ist.

6. Wärmflasche nach Anspruch 5. dadurch gekennzeichnet, daß die Beschichtung (3. 3') unterschiedliche Dicke und/oder Eigenschaften in verschiedenen Bereichen der Flasche (1) aufweist.

7. Wärmflasche nach Anspruch 5 oder 6, bei der die Beschichtung (3, 3') und/oder die Fasern wasserabweisende Eigenschaften besitzen.

8. Wärmflasche nach Anspruch 5 oder 6, bei der mindestens ein Teil (6, 7) der Flockbeschichtung (3, 3') lackiert ist.

9. Wärmflasche nach den Ansprüchen 5 bis 8, bei der der Körper der Flasche (1) aus thermoplastischem Material hergestellt ist.

10. Wärmflasche. wie sie durch ein Verfahren nach den Ansprüchen 1 bis 4 erhältlich ist.

## Revendications

1. Procédé pour la réalisation de bouillottes, caractérisé en ce qu'il comprend la phase de revêtement d'au moins une portion de la surface (4,5) de ladite bouillotte (1) par liaison à ladite surface d'une pluralité de fibres (3,3'), ayant dimensions prédetérminées, au moins à une partie de la surface de ladite bouillotte, par la technique dite de flockage.

2. Procédé suivant la revendication 1, caractérisé en ce qu'on fabrique ledit revêtement flocké (3, 3') avec caracteristiques différentes dans des zones différentes de la dite bouillotte.

3. Procédé suivant la revendication 1 ou 2, caractérisé en ce qu'on traite lesdites fibres, avant ou après leur application, avec un ou plusieurs produits imperméabilisants ou hydrofuges.

4. Procédé suivant la revendication 1 ou 2, caractérisé en ce qu'on peint au moins une partie (6, 7) de ledit revêtement flocké.

5. Bouillotte (1), caractérisée en ce qu'au moins une partie de sa surface (4, 5) est revêtue avec un revêtement (3, 3') formé par une pluralité de fibres de flock qui ont des dimensions prédetérminées et qui sont liées à ladite bouillotte par un moyen d'adhésion selon la technique dite de flockage.

6. Bouillotte suivant la revendication 5, caractérisée en ce que ledit revêtement (3, 3') a un épaisseur et/ou des caracteristiques différentes dans des zones différentes de ladite bouillotte (1).

7. Bouillotte suivant la revendication 5 ou 6, caractérisée en ce que ledit revêtement (3, 3') et/ou lesdites fibres ont des propriétés hydrofuges.

8. Bouillotte suivant la revendication 5 ou 6, caractérisée en ce qu'au moins une partie (6, 7) dudit revêtement flocké (3, 3') est peinte.

9. Bouillotte suivant l'une quelconque des revendications 5 à 8, caractèrisée en ce que le corps de ladite bouillotte (1) est fabriqué en matériel thermoplastique.

10. Bouillotte, obtenue par un procédé suivant l'une quelconque des revendications 1 à 4.
